# EUROPEAN PATENT APPLICATION

(11) **EP 0 546 697 A1**
(43) Date of publication of application: **16.06.1993**
(21) Application number: 92310491.3
(22) Date of filing: 18.11.1992
(51) Int. Cl.: C07B 39/00

(54) **Process for the bromination of deactivated toluenes in the benzylic position**

(30) Priority: 27.11.1991 US 799487
(71) Applicant: AT&T Corp., New York, NY 10013-2412 (US)
(72) Inventor: Chandross, Edwin Arthur, Berkeley Heights, New Jersey 07974 (US); Houlihan, Francis Michael, Millington, New Jersey 07946 (US); Neenan, Thomas Xavier, Jersey City, New Jersey 07302 (US); Schilling, Marcia Lea, Basking Ridge, New Jersey 07920 (US)
(74) Representative: Johnston, Kenneth Graham

(57) **Abstract**

Deactivated toluene compounds such as 2-nitro-6-trifluoromethyltoluenes, are brominated by stepwise addition of bromine at elevated temperatures. Subsequent conversion of the resulting brominated toluene to the corresponding benzyl alcohol allows esterification and formation of useful compounds such as photoacid generators.

## Description

### Background of the Invention

### 1. Field of the Invention

This invention relates to reactions involving toluene compounds and, in particular, to the bromination of toluene compounds.

### 2. Art Background

Many commercially significant materials are synthesized from toluene compounds, i.e. compounds including at least a methyl substituent on a phenyl ring. For example, as discussed in U.S. Patent 4,996,136, and U.S. patent application Serial No. 07/565,074, filed August 9, 1990, photoacid generators employed in resist materials for the manufacture of devices such as integrated circuits are made through a series of synthetic steps beginning with a toluene compound such as 2-nitro-6-trifluoromethyltoluene. The bromination of deactivated toluenes has applications in many other fields. Among these are: 1) the preparation of useful derivatives for agricultural chemicals based upon fluoroaromatics having deactivated toluene structures, such as 2-nitro-6-(trifluoromethyl)toluene (2-methyl-3-nitrobenzotrifluoride); 2) the preparation of useful derivatives for explosives based upon deactivated nitroaromatics such as 2,4,6-trinitrotoluene (TNT); 3) the preparation of sterically hindered o-nitrobenzyl derivatives useful as hydrolysis resistant photolabile protecting groups for functionalities such as carboxyl groups useful for the synthesis of intermediates leading to natural products. Typically, the beginning of these synthetic routes involves the bromination of the toluene, i.e. replacement of at least one active hydrogen in the methyl substituent on the phenyl ring. Generally, toluene brominations are accomplished by utilizing reagents such as N-bromosuccinimide (NBS) with radical initiators at temperatures not exceeding 100°C in solvents such as CCl₄. (See "Advanced Organic Chemistry", 3rd Ed. J. March 1985 p. 625 Wiley Interscience, N.Y.) Another approach involves use of molecular bromine at temperatures below 80°C. However, if the toluene is sterically hindered, that is, if substituents are present ortho to the methyl moiety and are approximately the same size or larger than trifluoromethyl, and/or if electron withdrawing groups, e.g. nitro, are present causing deactivation, bromination is difficult to achieve. Thus, for exemplary compounds like 2-nitro-6-trifluoromethyltoluene, conventional bromination reactions are not suitable and severe reaction conditions, such as use of molten NBS at high temperatures or use of stoichiometric concentrations of radical initiators having potential for violent reaction, are employed. However, these reactions are generally not acceptable in a commercial environment due to the potentially violent reaction conditions and/or solvents that have potential restrictions on use.

Various methods for brominating deactivated toluenes have been proposed for laboratory preparations. For example, as discussed by L. F. Fieser and W. E. Doering in J. of Am. Chem. Soc. 68, 2252, 1946, if the toluene material is sealed in a glass tube with bromine and magnesium carbonate and heated to a temperature at 160°C for 18 hours, bromination occurs even for deactivated compounds such as 2,4,6-trinitrotoluene. Although reaction proceeds, sealing a tube to prevent escape of the very volatile bromine is commercially inconvenient, and in the case of rapid reaction, possibly leads to detonation. Therefore, synthesis of commercially significant materials could be substantially improved if a more convenient method of brominating deactivated toluenes were available.

### Summary of the Invention

Deactivated toluenes such as 2-nitro-6-trifluoromethyltoluene, are advantageously brominated by the controlled, e.g. dropwise, addition of bromine to the toluene at a temperature typically above 180°C at ambient pressure, i.e. at approximately atmospheric pressure. Despite the volatility of the bromine and the relatively small amount present at any time during the reaction, high (greater than 80%) yield is achieved. This results in selective monobromination at the benzylic position. Additionally, by controlled addition, the required temperature is easily maintained and undesired reaction prevented. By using the resulting reaction product, such as through conversion to the corresponding alcohol and subsequent esterification, compounds such as bis(2-nitro-6-trifluoromethylbenzyl)-1-3-benzenedisulfonate (BBD) that are useful as photoacid generators are synthesized. Thus, esterification of 2-nitro-6-trifluoromethylbenzyl alcohol with 1,3-benzenedisulfonyl chloride yields the advantageous photoacid generator, bis(2-nitro-6-trifluoromethylbenzyl)-1,3-benzenedisulfonate.

### Detailed Description

The bromination of the deactivated toluene compound is accomplished by controlled addition, e.g. dropwise addition of elemental bromine to the deactivated toluene material at temperatures greater than 150°C and at atmospheric pressure. This addition is achieved by expedients such as dropwise introduction of the bromine through a Teflon® tube that extends below the surface of the toluene material. It is generally convenient to add the bromine as a solution in the toluene if the deactivated toluene material is a liquid. Many toluene materials such as 3,5-dinitro-4-toluic acid are liquids at the reaction temperature, and thus, bromine is easily added directly to the deactivated toluene material. For materials that are solids at the reaction temperature, the reaction occurs in solution provided the solvent is chosen to be relatively inert to the bromine reagent.

Generally, for typical deactivated toluene reactants, bromine addition rates of between 0.3 and 1 mole percent of molecular bromine per minute are employed to maintain a suitably high temperature for the bromination reaction to proceed. (The mole percentage is that of the total stoichiometric amount of molecular bromine to be added.) The rate of molecular bromine addition should be sufficiently slow to avoid accumulation of unreacted bromine. That is, the bromine addition should generally not be so rapid that the temperature is lowered sufficiently to prevent reaction for a total period of more than 1 hour over the course of the benzylic bromination. Thus, rates less than 0.3 mole percent per minute are not precluded but generally are inconveniently slow. If excessive free bromine is allowed to build up, the bromine vapor refluxes, cooling the reaction mixture to a temperature at which the reaction rate is substantially decreased, i.e., decreased by an order of magnitude from the rate achieved at 180°C. The reaction is reinitiated (if necessary) by allowing the refluxing bromine to react and the temperature to rise, substantially reducing the danger of runaway reaction in this self-quenching system.

The reaction is advantageously employed with deactivated toluenes, i.e. substituted toluenes where the methyl substituent is adjacent to a group having a Charton steric parameter greater than or equal to 0.92 and/or electron withdrawing groups having a sigma constant greater than 0.35. (Charton parameter and sigma constant are defined in "Substituent Constants for Correlation Analysis in Chemistry and Biology", C. Hansch, A. Leo, Wiley Interscience, N.Y. 1979, p.1-8 (electronic), p.9-12 (steric).) Although electronic effects associated with electron withdrawing groups do affect the reaction, generally the greatest influence on reaction rate and required temperature of reaction is the size of a substituent at an ortho position providing steric hindrance. Thus, the required temperature to initiate reactions varies with the particular substituents. However, temperatures below the decomposition temperature of reactants and products in the range of 150°-200°C, are generally suitable. A control sample is easily employed to determine a useful reaction temperature for a specific desired reactant.

The reaction mixture, in the absence of solvent, contains the desired brominated material, HBr, and to an extent, unreacted bromine. The HBr and the bromine are easily removed by purging the cooled mixture with an inert gas such as nitrogen, and the resulting purged material is purified typically by distillation or recrystallization.

The resulting product: 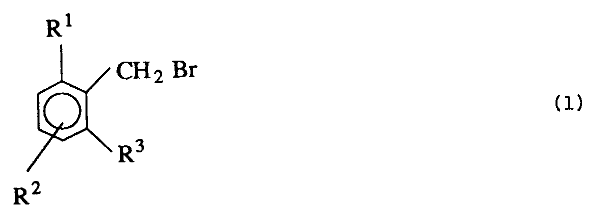
where R¹ and R³ are substituents providing deactivation, either by steric and/or electronic effects and R² is a substituent such as one providing deactivation by electronic effects, is then advantageously employed as an intermediary in making useful materials such as photoacid generators where, for example, R¹ and/or R³ is NO₂. (If R² provides substantial electronic deactivation, then either R¹ or R³, but not both, need not be present for advantageous use of the invention. Additionally, it is contemplated that if R² is H or another substituent that does not provide deactivation, R¹ and/or R³ should be present for deactivation and thus advantageous use of the invention.) A typical reaction sequence for making materials such as photoacid generators, involves the conversion of the brominated material to a corresponding alcohol, 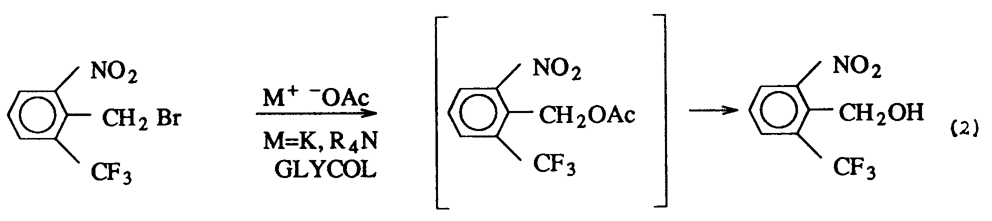
This conversion is advantageously accomplished using an acid salt whose parent acid has a pKₐ typically in the range of 4 to 6 to form a carboxylate ester as an intermediate which subsequently converts to the alcohol. The counterion in the salt, if ultimate use is for applications such as semiconductor device manufacture where metal impurities are not desirable, should be a cation such as a tetraalkylammonium entity. For example, lower tetraalkylammonium cations, e.g. methyl and ethyl, are advantageously employed. Ammonium entities with larger alkyl, e.g. butyl, substituents are also useful but react somewhat more slowly. Typical acids are lower alkane acids, such as the propionic or acetic. Generally, the reaction is done in a solvent that does not undergo undesirable side reactions with the reagents. Typical solvents, such as ethylene glycol or propylene glycol, are advantageously employed. Generally, the reaction is performed at 95-105°C and affords a yield in excess of 80%. After formation of the ester, the glycol (or other material providing a hydroxyl group) removes the acyl group to form the desired benzyl alcohol.

The resulting reaction product is typically purified by introduction into a medium such as water, to remove by dissolution the solvent and byproduct salts. The resulting crystals are generally carefully dried since subsequent reactions are often sensitive to the medium utilized to remove a byproduct. The alcohol of equation (2) is then converted to the desired product by conventional reactions such as esterification. Such esterification is typically accomplished by reaction with an acid chloride in the presence of an acid acceptor. Acid chlorides such as 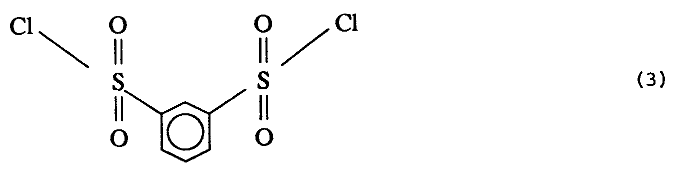
are typically used to make products such as photoacid generators. The reaction is typically done in an acetone or ethyl acetate medium at a temperature in the range 0 to 15°C. Exemplary of suitable acid chlorides, are 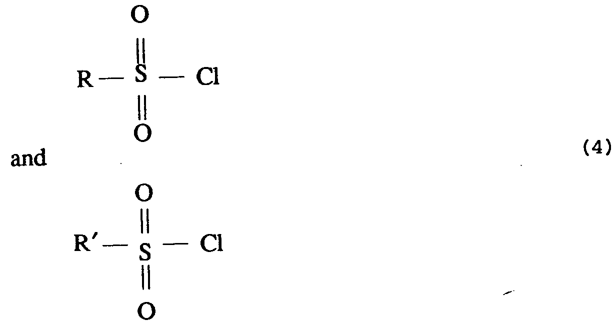
where R is lower alkyl, or substituted lower alkyls such as 2,2,2-trifluoroethyl and where R' is an entity such as phenyl or substituted phenyl, e.g., p-nitrophenyl or trifluoromethylphenyl. Generally, for sulfonic acid chlorides that are suitable for preparation of a photoacid generator, the corresponding sulfonic acid should be substantially more acidic in the resist-polymer matrix than p-toluenesulfonic acid.

The following examples are illustrative of conditions utilized in the inventive process.

### Example 1

A 250 mL three-neck flask was fitted with a magnetic stir bar, condenser (0°C), thermocouple (in a well) and a constant addition funnel. After purging the flask assembly for 1 h with N₂, the inlet adapter was replaced with tubing connected in sequence to a CaCl₂ drying tube, a trap and a scrubber (1.5 L 25% aqueous NaOH).

The reaction was carried out using 80.0 g (0.40 mol) of 2-nitro-6-trifluoromethyltoluene; 60 g was added to the flask initially and remaining 20 g was used to dissolve the molecular bromine (57.6 g, 0.36 mol). The flask assembly was lowered into a preheated oil bath (180°C) and allowed to equilibrate. The temperature of the oil bath was then gradually raised to 195°C in order to raise the pot temperature to 180°C. The molecular bromine mixture was added dropwise (added under the liquid level via a TEFLON® transport tube) at a rate to maintain the pot temperature at 180°C and to minimize evaporation from the reaction mixture.

Aliquots were removed periodically to monitor the progress of the reaction by ¹H NMR. When 80-90% of the molecular bromine mixture had been added, there was some refluxing which cooled the flask temperature to 172°C. NMR indicated 90% conversion to the benzyl bromide. The entire system was purged with N₂ into the scrubber solution to remove residual HBr and molecular bromine before distillation to give pure 2-nitro-6-trifluoromethylbenzyl bromide (b.p.80-82°C, 0.05 mm Hg). Anal Calcd: C: 33.80 H: 1.76 N: 4.93; Found C: 33.85 H: 1.89 N: 4.99; ¹H NMR (ppm CDCl₃) 8.14-7.17 (m, 3H) 4.79 (s, 2H); IR (cm⁻¹ film NaCl) 3020, 1570, 1510, 1395, 1304, 1160, 1120, 895, 825.

### Example 2

A 250 mL three necked flask equipped with a thermocouple, addition funnel and DRY ICE® condenser was charged with 3,5-dinitro-4-toluic acid (20.0 g, 88.5 mmole). The reaction flask, under argon, was then immersed into an oil bath which was heated to 210°C (the reaction mixture became molten at approximately 190°C). Bromine (14.1 g, 4.54 mL, 88.5 mmoles) was added through a Teflon® tube immersed into the molten reaction mixture. The addition of bromine was done slowly to ensure that it reacted completely as added; otherwise, a refluxing of bromine lowered the temperature of the melt below that needed for effective reaction (∼170-180°C). The addition was complete after about 5 h, and the cooled reaction mixture was flushed with nitrogen overnight to remove HBr and unreacted bromine. The fused reaction mass was then broken into a powder in a mortar and pestle, and dried under vacuum 2 h. In this way, crude 4-(hydroxycarbonyl)-2,6-dinitrobenzyl bromide (77 mole percent desired compound, remainder unreacted dinitrotoluic acid) (21.0 g, 54% yield) was obtained. ¹H NMR (ppm, dimethylsulfoxide-d₆) 12.5(broad s, 1H); 4.87(s, 2H); 8.70(s, 2H). IR(cm⁻¹, NaCl film); 1690, 1530,1350.

### Example 3

A 200 mL reaction vessel equipped with a magnetic stirrer, reflux condenser and a nitrogen inlet was charged with 2-nitro-6-trifluoromethylbenzyl bromide (8.0 g, 28 mmol). A solution of tetramethylammonium acetate (TMAA) in propylene glycol (Southwestern Analytical) (20% by weight, 25 g) was added. (The weight of contained TMAA is 5.0 g, 38 mmol, 1.3 equivalents of acetate per bromide). The reaction vessel was immersed in an oil bath whose temperature was maintained between 95-100°C. A pale yellow homogeneous solution formed immediately. The reaction mixture was heated for a total of 28 h, with 0.1 mL aliquots being removed periodically and analyzed by thin layer chromatography (TLC) (SiO₂, methylene chloride). The reaction mixture was cooled and poured into rapidly stirred water (5 volumes). A dark oil immediately precipitated which solidified upon further stirring (usually within 10-15 minutes). The mixture was cooled to 5°C and filtered. The yellow/brown solid was broken up with a spatula and washed further with 200 mL of 5°C water, followed by drying in vacuo. The yield (average of 4 runs) was 81 %. Thin layer chromatography of this material showed it to be one compound with a residue at the baseline. Recrystallization from pentane/methylene chloride removes the residue. Recrystallized product yields were in the 75% range. M.P. 68°C. IR (film on NaCl) 3300 (broad), 2990 (m), 1540 (s), 1370 (vs). ¹H NMR (CDCl₃) 8.12-7.63 (m, 3H), 4.92 (d, 2H), 2.92 (bs, 1H). Elemental Analysis: Calcd. C: 43.44, H: 2.71, N: 6.33, Found C: 43.34, H: 2.81, N: 6.43.

### Example 4

A solution was prepared under argon consisting of m-benzenedisulfonyl chloride (7.09 g, 25.78 mmole) and 2-nitro-6-trifluoromethylbenzyl alcohol (12.00 g, 54.27 mmole) in dry ethyl acetate (40 mL). To this solution was added, dropwise over almost 20 min, dicyclohexylamine (9.84 g, 54.3 mmole) in ethyl acetate (15 mL), keeping the temperature of the reaction mixture below 15°C (∼ 10°C). The reaction was followed by TLC (using CH₂Cl₂ as solvent) and was complete after stirring 2 hours at 10°C after final addition of the amine. The precipitated hydrochloride salt was removed by filtration, and washing the precipitate with about 70 mL of ethyl acetate to make sure all of the product was removed (testing done by TLC). The combined ethyl acetate solution (125 mL) was diluted with an equal volume of hexane. This solution was passed through a silica gel column (35 cm long with 40 g of silica gel), using ethyl acetate/hexane 1:1 as the eluent. The filtrate and washing were combined (400 mL) to which was added hexane until persistent cloudiness was obtained. This solution was cooled immediately in ice. Crystals precipitated out within a minute and additional hexane was added until the solution was no longer cloudy. It was then filtered (14.70 g, 88%). The reprecipitation was repeated to give clean material. (13.50 g, 81%). M.p. 100°C. Elemental analysis: Calcd C: 41.00, H: 2.19, N: 4.34; Found C: 40.89, H: 2.23, N: 4.21. IR (film on NaCl) 1543 (vs), 1374 (vs), 1314 (vs), 1200 (s), 1185 (vs), 1135 (vs), 1100 (s), 960 (vs), 860 (s), 835 (s), 745 (s), 685 (s). ¹H NMR (CDCl₃) 5.53 (s, 4H), 8.40 (t, J=1.18 Hz, 1H), 8.29 (t, J=1 Hz, 1H), 8.18 (d, J=1.8 Hz, 1H), 8.03 (t, J=1.8 Hz, 1H), 7.94 (d, J=1.8 Hz, 3H), 7.85-7.61 (m, 3H).

## Claims

1. A process for fabricating a material including the step of brominating a deactivated toluene in the benzylic position, said brominating reaction comprising the steps of combining said bromine with said deactivated toluene at elevated temperature, characterized in that said bromine is added incrementally at substantially atmospheric pressure after reaction initiation.

2. The process of claim 1 wherein said reaction is performed at a temperature in the range 150°C to 200°C.

3. The process of claim 1 wherein said brominated deactivated toluene is represented by the formula: 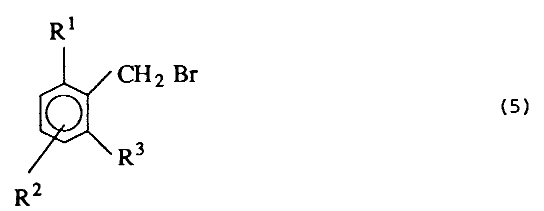 where R¹, R², and R³ are individually hydrogen or substituents that provide deactivation through steric or electronic effects such that the net effect of said substituents is a deactivated toluene.

4. The process of claim 3 wherein R¹ is NO₂.

5. The process of claim 4 wherein R³ is NO₂ or trifluoromethyl.

6. The process of claim 1 including the step of reacting said brominated toluene with a carboxylic acid salt and subsequent conversion to form the corresponding alcohol.

7. The process of claim 6 wherein said salt comprises a tetraalkylammonium salt.

8. The process of claim 6 including the step of reacting said alcohol with an acid chloride.

9. The process of claim 8 wherein said acid chloride comprises a sulfonyl chloride.

10. The process of claim 9 wherein said acid chloride comprises
